# EUROPÄISCHE PATENTSCHRIFT

(11) **EP 0 339 382 B1**
(45) Veröffentlichungstag und Bekanntmachung des Hinweises auf die Patenterteilung: **29.06.1994**
(21) Anmeldenummer: 89106596.3
(22) Anmeldetag: 13.04.1989
(51) Int. Cl.: C11C 1/08, C11B 1/02

(54) **Verfahren zur Desodorierung von Fettsäureestergemiscchen**
Process for deodorizing mixtures of fatty acid esters
Procédé pour désodoriser des mélanges d'esters d'acides gras

(30) Priorität: 23.04.1988 DE 3813805
(43) Veröffentlichungstag der Anmeldung: 02.11.1989
(73) Patentinhaber: BASF Aktiengesellschaft, 67063 Ludwigshafen (DE)
(72) Erfinder: Hoercher, Ulrich, Dr., D-6800 Mannheim 1 (DE); Lechtken, Peter, Dr., D-6710 Frankenthal (DE)

(56) Entgegenhaltungen:
- US-A- 2 380 408
- US-A- 3 984 479

## Beschreibung

Die Erfindung betrifft ein Verfahren zur Entfernung von unerwünschten Gerüchen aus Fettsäureestergemischen, die neben anderen Komponenten die Ester von hochungesättigten Fettsäuren, insbesondere die Ester der hochungesättigten Fettsauren Eikosapentaensäure (EPA) und Dokosahexaensäure (DHA) enthalten.

Die zur Gruppe der omega-3-Fettsäuren gehörenden Verbindungen EPA und DHA sind kürzlich als Mittel zur Verhinderung von Thrombose und Arteriosklerose und zur Senkung des Triglyceridspiegels bekannt geworden (vgl. "Ungesättigte Fettsäuren in der Prophylaxe und Therapie" in Apothekerjournal 10 (1) (1988) S. 38-44) sowie loc. cit. 10(2) S. 48-52). Sie treten vor allem in Fischölen und den Ölen und Fetten von Algen auf. Fischöle mit Gehalten an EPA und DHA von 20 - 30 % werden zur Aufreinigung dieser Verbindungen und der Herstellung von diätetischen Lebensmitteln verwendet. In diesen EPA- und DHA-haltigen Präparaten können die Fettsäuren unter anderem als Triglyceride oder als Ester niederer Alkohole vorliegen. Die durch Umesterung mit niederen Alkanolen aus den Triglyceriden erhaltenen Alkylester haben den Vorteil, daß sie besonders für die Aufkonzentrierung von EPA und DHA geeignet sind. Verfahren zur Umesterung von Fischölen mit niederen Alkanolen und die anschließende Aufkonzentrierung von EPA und DHA sind bereits mehrfach beschrieben (vgl. E.J. Gauplitz et al in J. Am. Oil Chem. Soc. 40 (1963) S. 197-198 bzw. US-A-4,377,526).

Ein Problem bei der Herstellung von EPA- und DHA-haltigen Präparaten aus Fischölen für die orale Einnahme ist der Fischgeruch, der auch nach Umesterung und Aufkonzentrierung von EPA und DHA äußerst hartnäckig erhalten bleibt. Dieser Geruch ist auf eine Vielzahl von Verbindungen zurückzuführen, die durch oxidativen Abbau hochungesättigter Fettsäuren entstehen (vgl. J. Amer. Oil Chem. Soc. 52 (1975) Seiten 349-53). Dabei handelt es sich überwiegend um ungesättigte Carbonylverbindungen, die schon bei sehr niedrigen Konzentrationen intensiv riechen können.

Die Entfernung von Geruchsstoffen aus Triglyceriden ist durch Desodorierung mit Wasserdampf unter vermindertem Druck möglich. Für Fettsäurealkylester ist dies Verfahren weniger gut geeignet, da aufgrund ihrer höheren Flüchtigkeit ein Teil der Ester mit dem Wasserdampf überdestilliert.

In vielen Fischölpräparaten wird das Geruchs- und Geschmacksproblem durch Verkapselung des Öls in Weichgelatinekapseln gelöst. Dadurch wird jeder Kontakt mit dem Öl bei der Einnahme vermieden. Doch auch hier kann sich nachträglich der Fischgeschmack (z.B. beim Aufstoßen) unangenehm bemerkbar machen. Es ist also in jedem Fall erstrebenswert, unabhängig von der gewählten Formulierung, ein möglichst geruchloses Öl zu verwenden.

Eine weitere Möglichkeit zur Herstellung geruch- und geschmackloser Fischölpräparate ist die Bildung von Cyclodextrin-Einschlußverbindungen (vgl. DE-A-3 226 232 und DE-A-3 419 796). Hierbei wird ein geruchloses stabiles Trockenpulver erhalten. Der Nachteil dieser Methode besteht in der Erhöhung der einzunehmenden Menge um über 100 % durch das Trägermaterial.

Es war daher die Aufgabe der Erfindung ein Verfahren zur Entfernung unerwünschter Gerüche aus hochungesättigte Fettsäuren enthaltenden Fettsäureestergemischen, insbesondere aus hochungesättigte omega-3-Fettsäurealkylester enthaltenden Fettsäureestergemischen, wie aus EPA- und DHA-haltigen Fettsäurealkylestergemischen zu finden, das die Nachteile der zuvor genannten bekannten Verfahren nicht aufweist.

Es wurde nun überraschenderweise gefunden, daß eine solche Desodorierung durch Ausrühren der Estergemische mit einer Lösung komplexer Hydride, wie einer wäßrigen Natriumborhydridlösung auf einfache Weise möglich ist.

Hierbei werden die den Fischgeruch verursachenden Carbonylverbindungen reduziert. Mit der Abnahme des Fischgeruchs tritt gleichzeitig ein leicht fruchtiger Geruch auf, der von den reduzierten Carbonylverbindungen stammt.

Gegenstand der Erfindung ist daher ein Verfahren zum Entfernen von unerwünschten Gerüchen aus Fettsäureestergemischen, die neben anderen Komponenten die Ester von hochungesättigten Fettsäuren enthalten, dadurch gekennzeichnet, daß man die Fettsäureestergemische mit komplexen Hydriden behandelt.

Besondere Bedeutung hat das Verfahren für die Entfernung von unerwünschten Gerüchen aus Fettsäureestergemischen, die Ester von hochungesättigten omega-3-Fettsäuren, insbesondere von Eicosapentaensäure und/oder Docosahexaensäure, enthalten. Das Verfahren ist anwendbar für Gemische, die die hochungesättigten Fettsäuren als Triglyzeride enthalten, hat aber besondere Bedeutung für Gemische, die die hochungesättigten Fettsäuren als Ester von Alkanolen mit 1 bis 3 C-Atomen enthalten, insbesondere für solche Gemische, die Ester von hochungesättigten omega-3-Fettsäuren und Alkanolen mit 1 bis 3 C-Atomen enthalten. Mit besonderem Vorteil verwendet man Fettsäureestergemische, die durch Umesterung von Fischöl mit Alkanolen mit 1 bis 3 C-Atomen erhalten wurden, insbesondere solche, in denen die Ester von EPA und/oder DHA vorher angereichert wurden.

Als komplexe Metallhydride bezeichnen wir erfindungsgemäß Verbindungen, wie Lithiumaluminiumhydrid, Natrium- und Lithiumborhydrid, sowie LiAlH[OC(CH)₃]3 in THF und Diisoamylboran in THF, insbesondere Lithiumaluminiumhydrid und Natriumborhydrid. Bei der Umsetzung mit LiAlH₄ muß in wasserfreiem Medium, beispielsweise in Diethylether, gearbeitet werden. Mit besonderem Vorteil arbeitet man mit NaBH₄. Da es sich in Wasser nur sehr langsam zersetzt, kann man mit wäßriger NaBH₄-Lösung arbeiten, was verfahrenstechnisch große Vorteile bietet.

Die Konzentration der Natriumborhydridlösung kann 0,01 - 10 %, bevorzugt 0,1 - 0,5 % betragen. Fischöle, die als Ausgangsstoffe für die Herstellung der Ethylestergemische dienen, können als Naturprodukte unterschiedlicher Herkunft und Verarbeitung unterschiedliche Konzentrationen an Geruchsstoffen besitzen, Dies gilt auch für die daraus hergestellten Ester. Daher ist die zum Erreichen des gewünschten Desodoriereffekts erforderliche Konzentration der Natriumborhydridlösung nicht genau vorhersagbar, im allgemeinen sind aber 0,1 - 0,5 gew.% ige Lösungen ausreichend.

Bezogen auf das Estergemisch werden 0,1 bis 5 Teile, bevorzugt 0,5 - 2 Teile der Natriumborhydridlösung eingesetzt, Die Reaktionstemperatur beträgt 20 - 60°C. Das Reaktionsgemisch bildet unter leichtem Schäumen eine Emulsion, Durch Ansäuern mit verdünnter Schwefelsäure nach beendeter Reaktion läßt sich diese Emulsion brechen, Anschließend wird die Esterphase mehrmals mit Wasser gewaschen, bis das Waschwasser neutral ist. Sollten bei diesen Phasentrennungen Emulsionen auftreten, können diese mit Ethanol oder Natriumchloridlösung beseitigt werden.

Die so behandelten Fettsäureester besitzen statt des fischartigen einen fruchtig-aromatischen Geruch. Sie können nach allen bekannten Methoden weiter stabilisiert und formuliert werden, z.B. durch Zugabe von Anti-oxidantien, Weichgelatineverkapselung, Mikroverkapselung oder Herstellung von Emulsionen.

Die folgenden Beispiele sollen die Erfindung näher illustrieren, ohne sie zu beschränken.

### Beispiel 1

300 g eines durch Umesterung von Sardinenöl mit Ethanol in Gegenwart von Natriumethylat erhaltenen Fettsäureethylestergemisches mit einem Gehalt von 13 % EPA und 9 % DHA wurden 30 Minuten (Min) lang bei 40°C mit 300 ml einer 0,1 Gew.%igen Lösung von Natriumborhydrid in Wasser gerührt, wobei sich unter leichtem Schäumen eine Emulsion bildete. Nach Zugabe von 100 ml 3%-iger Schwefelsäure trennten sich die Phasen. Die organische Phase wurde 3 mal mit 200 ml Wasser gewaschen, wobei man ggf. auftretende Emulsionen durch Zugabe von wenig Ethanol oder Natriumchloridlösung beseitigte. Wasser- und Lösungsmittelreste wurden bei 40°C und 1 mbar destillativ entfernt. Man erhielt 295 g eines Estergemisches, dessen fischartiger Geruch weitgehend verschwunden war, und der dafür fruchtartig-aromatisch roch.

Das Produkt wurde in 2 Hälften geteilt (A und B). Probe A wurde luftdicht unter Stickstoff, B dagegen unter Luftkontakt aufbewahrt. Nach 10 Tagen hatte Probe A den fruchtigen Geruch beibehalten, während Probe B einen leinölartigen Geruch angenommen hatte.

### Beispiel 2

200 g eines kommerziell erhältlichen Konzentrates aus omega-3-Fettsäureethylestern (30 % EPA, 20 % DHA) wurden 30 Min. bei 40°C mit 200 ml einer 0,1 Gew.% igen Natriumborhydridlösung gerührt. Nach Aufarbeitung analog Beispiel 1 hatte das Produkt einen aromatischen Geruch angenommen.

### Beispiel 3

6 ml einer technischen Natriumborhydridlösung (Fa. Ventron, 12,5 % NaBH₄ in 40 gew.% iger Natronlauge) wurden auf 300 ml verdünnt. Mit dieser Lösung wurde wie in Beispiel 1 beschrieben eine Desodorierung durchgeführt, die zum gleichen Erfolg führte.

### Beispiel 4

100 g eines durch Umesterung von Fischöl und Aufkonzentrieren durch Behandeln mit Harnstofflösung und anschließendes Destillieren gemäß dem Verfahren nach US 4,377,526 hergestellten Ethylesterkonzentrats enthaltend 34% EPA und 21% DHA wurden mit 100 g einer 0,25 gew.%igen Natriumborhydridlösung 30 Min. bei 40°C gerührt und anschließend mit 100 ml 10%ige Schwefelsäure versetzt. Nach Abtrennung der wäßrigen Phase, 3-maligem Waschen der Ethylester mit 100 ml Wasser und destillativem Entfernen von Wasserresten unter vermindertem Druck erhielt man ein leicht fruchartig riechendes Produkt.

## Patentansprüche

1. Verfahren zum Entfernen von unerwünschten Gerüchen aus Fettsäureestergemischen, die neben anderen Komponenten die Ester von hochungesättigten Fettsäuren enthalten, dadurch gekennzeichnet, daß man die Fettsäureestergemische mit komplexen Hydriden behandelt.

2. Verfahren gemäß Anspruch 1, dadurch gekennzeichnet, daß man Fettsäureestergemische einsetzt, die Ester von hochungesättigten ω-3-Fettsäuren enthalten.

3. Verfahren gemäß Anspruch 1, dadurch gekennzeichnet, daß man Fettsäureestergemische einsetzt, die Ester von Eicosapentaensäure (EPA) und/oder Docosahexaensäure (DHA) enthalten.

4. Verfahren gemäß Anspruch 1, dadurch gekennzeichnet, daß man die Fettsäureestergemische mit einer Natriumborhydridlösung behandelt.

5. Verfahren gemäß Anspruch 1, dadurch gekennzeichnet, daß man Gemische von Estern aus hochungesättigten Fettsäuren und Alkanolen mit 1 bis 3 C-Atomen einsetzt.

6. Verfahren gemäß Anspruch 1, dadurch gekennzeichnet, daß man Gemische von Estern aus hochungesättigten ω-3-Fettsäuren und Alkanolen mit 1 bis 3 C-Atomen mit einer Natriumborhydridlösung behandelt.

7. Verfahren gemäß Anspruch 1, dadurch gekennzeichnet, daß man Fettsäureestergemische verwendet, die durch Umesterung von Fischöl mit Alkanolen mit 1 bis 3 C-Atomen erhalten wurde.

8. Verfahren gemäß Anspruch 1, dadurch gekennzeichnet, daß man Fettsäureestergemische verwendet, in der die Ester von Eicosapentaensäure und/oder Docohexaensäure vorher angereichert wurden.

## Claims

1. A process for removing undesirable odors from a fatty ester mixture which, in addition to other components, contains the esters of highly unsaturated fatty acids, wherein the fatty ester mixture is treated with a complex hydride.

2. A process as claimed in claim 1, wherein the fatty ester mixture used contains esters of highly unsaturated ω-3-fatty acids.

3. A process as claimed in claim 1, wherein the fatty ester mixture used contains esters of eicosapentaenoic acid (EPA) and/or docosahexaenoic acid (DHA).

4. A process as claimed in claim 1, wherein the fatty ester mixture is treated with a sodium borohydride solution.

5. A process as claimed in claim 1, wherein a mixture of esters of highly unsaturated fatty acids and alkanols of 1 to 3 carbon atoms is used.

6. A process as claimed in claim 1, wherein a mixture of esters of highly unsaturated ω-3-fatty acids and alkanols of 1 to 3 carbon atoms is treated with a sodium borohydride solution.

7. A process as claimed in claim 1, wherein the fatty ester mixture used has been obtained by transesterification of fish oil with an alkanol of 1 to 3 carbon atoms.

8. A process as claimed in claim 1, wherein the fatty ester mixture used is one in which the esters of eicosapentaenoic acid and/or docosahexaenoic acid have been concentrated beforehand.

## Revendications

1. Procédé pour éliminer les odeurs indésirables de mélanges d'esters d'acides gras qui contiennent, en plus d'autres composants, des esters d'acides gras à haut degré d'insaturation, caractérisé en ce que l'on traite les mélanges d'esters d'acides gras par des hydrures complexes.

2. Procédé selon la revendication 1, caractérisé en ce que l'on met en réaction des mélanges d'esters d'acides gras qui contiennent des esters d'acides gras ω-3 à haut degré d'insaturation.

3. Procédé selon la revendication 1, caractérisé en ce que l'on met en réaction des mélanges d'esters d'acides gras qui contiennent des esters d'acide eicosapentaénoïque (EPA) et/ou d'acide docosahexaénoïque (DHA).

4. Procédé selon la revendication 1, caractérisé en ce que l'on traite les mélanges d'esters d'acides gras par une solution de borohydrure de sodium.

5. Procédé selon la revendication 1, caractérisé en ce que l'on met en réaction des mélanges d'esters d'acides gras à haut degré d'insaturation et d'alcanols à 1-3 atomes de carbone.

6. Procédé selon la revendication 1, caractérisé en ce que l'on traite des mélanges d'esters d'acides gras ω-3 à haut degré d'insaturation et d'alcanols à 1-3 atomes de carbone par une solution de borohydrure de sodium.

7. Procédé selon la revendication 1, caractérisé en ce que l'on utilise des mélanges d'esters d'acides gras qui ont été obtenus par transestérification d'huile de poisson avec des alcanols à 1-3 atomes de carbone.

8. Procédé selon la revendication 1, caractérisé en ce que l'on utilise des mélanges d'esters d'acides gras qui ont été préalablement enrichis en les esters d'acide eicosapentaénoïque et/ou d'acide docosahexaénoïque.
